# EUROPEAN PATENT SPECIFICATION

(11) **EP 1 321 136 B1**
(45) Date of publication and mention of the grant of the patent: **14.12.2011**
(21) Application number: 02291542.5
(22) Date of filing: 20.06.2002
(51) Int. Cl.: A61K 8/11, A61K 8/67, A61K 8/81, A61Q 19/00, A61Q 19/08, A61K 8/02

(54) **Cosmetic compositions of powder-type containing anti-wrinkling ingredients**
Kosmetische Zusammensetzungen vom Pulvertyp mit Wirkstoffen gegen Hautfalten
Compositions cosmétiques de type pulvérulent contenant des ingrédients anti-rides

(30) Priority: 20.12.2001 KR 2001081822
(43) Date of publication of application: 25.06.2003
(73) Proprietor: Coreana Cosmetics Co., Ltd., Cheonan-si, Chungcheongnam-do (KR)
(72) Inventor: Kim, Dong-Myung, Suwon-Si, Kyoungki-Do (KR); Jo, Byoung-Kee, Anyang-Si, Kyoungki-Do (KR); Kim, Hyo-Yong, Kwangju-Si (KR)
(74) Representative: De Souza, Paula

(56) References cited:
- WO-A-01/35933
- US-A- 5 585 090

## Description

### BACKGROUND OF THE INVENTION

### Field of the Invention

The present invention relates to powder-type cosmetic compositions which contain a stabilized anti-wrinkling ingredient effective for reducing skin wrinkles, and more particularly to powder-type cosmetic compositions having little skin irritant effect and excellent anti-wrinkling effect in the form of a powder containing a large amount of liquid phase ingredient.

### Description of the Related Art

During childhood, the skin rapidly recovers from damage caused by sunlight. But, as we get older, wrinkles, calluses, sagging, inelasticity, roughness, dryness, mottling, etc., of the skin by exposure to sunlight are likely to happen. The general term for these changes is "photo aging". Namely, photo aging damages the epidermal tissue and hypodermal tissue of the skin. Also, it causes wrinkles to form, and skin to turn yellowish, thick and inelastic.

Recent industrial and economical development have 1ed to increased number of vehicles such as automobiles, and household appliances such as heaters and air-conditioners. As such, air pollution and noise pollution are growing more and more serious. Pollutants from automobiles, particularly nitrogen compounds, contain ozone-depleting material and thus destroy the ozone layer. In addition to these pollutants, water pollution and soil pollution are increasing sharply. Also, allergenic sensitization to pollen in spring and fall is a cause of contact dermatitis.

Many skin care cosmetics to protect the skin against photo aging and various pollutants as mentioned above have been developed. Cosmetic for reducing wrinkles, cosmetics for skin whitening and cosmetics for protection from the sun can be exemplified.

Examples of cosmetic formulations which can impart moisturizing, anti-wrinkling or whitening effect to the skin are typical skin care formulations such as after shave, lotions and creams, and makeup formulations for beautifying the skin by coloring the skin and for masking skin troubles such as melasma and ephelides. Among these, powder-type cosmetic formulations containing liquid phase ingredient such as anti-wrinkle or whitening active ingredients are known.

Japanese Patent Laid-Open Nos. 6-166611 and 5-65212 describe powder-type cosmetic compositions containing a large amount of liquid phase ingredient, comprising a hydrophobized silica having specific surface area of 80 m²/g or more, a powder surface-treated with a fluorine compound, an oily ingredient and an aqueous ingredient (30-90%). These compositions can absorb water using an amorphous silica. But, on account of the high hydrophilicity of the powder surface, formulation into powder is very difficult. So, the amorphous silica is appropriately hydrophobic-treated by a silicone compound and a fluorine compound in these applications.

However, the compositions have disadvantages that they are difficult to formulate homogeneously when the liquid phase ingredient is added in a heterogeneous mixing state with the powder or the mixing speed of a mixer is not constant. Also, the powders tend to aggregate during the production of the cosmetic compositions. Particularly, it is difficult to mix the powder with an active ingredient such as retinol and its derivatives, etc., and titer and stability of the compositions are not maintained for a long time.

Examples of the Retinol derivatives include retinal (vitamin A aldehyde), retinyl acetate, retinyl propionate, retinyl linoleate, etc. However, retinol and its derivatives, though believed to cause beneficial skin effects, e.g., anti-wrinkling effect, has never been successfully formulated in the stable form.

Many efforts to stable the retinal and its derivatives using some compounds in the cosmetic compositions have been made.

U.S. Pat. No. 3,906,108 discloses an emulsion (O/W type) in which retinoic acid is stabilized by BHT{2,6 bis(1,1-dimethylethyl)-4-methoxyphenol} as an antioxidant, dl-α-tocopherol and EDTA as a chelating agent.

U.S. Pat. No. 4,247,547 discloses an emulsion (O/W type) in which retinol is stabilized by tocopherol as an antioxidant and citric acid.

U.S. Pat. No. 4,826,828 discloses an emulsion (W/O type) in which retinal, retinyl acetate and retinyl palmitate are stabilized by BHT and BHA (butyllated hydroxyanisole) as antioxidants.

U.S. Pat. No. 4,720,353 discloses an emulsion (W/O type) in which retinol is stabilized by BHA as an antioxidant, ascorbic acid and tocopheryl linoleate.

European Pat. No. 0 440 398 B1 discloses an emulsion (O/W type) composition which comprises at least one water-soluble antioxidant, at least one antioxidant and a chelating agent in order to chemically stabilize retinoid.

European Pat. No. 1 568 106 A1 discloses an emulsion (O/W type) composition which comprises a chelating agent and an antioxidant, a chelating agent and a water-soluble antioxidant, and an antioxidant capable of existing in an oily phase and an aqueous phase of an emulsion and imidazole of a free base type, as a system for stabilizing retinoid.

WO03035016 discloses polymeric particles chosen from the methylsilsesquioxane resin microspheres and additional ingredients such as retinoid derivatives such a as retinyl palmitate or retinyl propionate, tocopherol acetate, further antioxidants. The document teaches dimethicone copolyol as emulsifier and various polymer powders selected form vinyl dimethicone crosspolymers, dimethicone copolyol or cyclomethicone and absorbent silica powder but no polymethylmethacrylate encapsulation.

WO0137800 discloses powder-to-liquid compositions comprising coated silica powder, resin particles of HDI/trimethylyol hexyllactone crosspolymer, polymethylsilsesquioxane particles, PMMA powder, and optionally titanium dioxide/dimethicone, antiwrinkle agent and antioxidant but no polyol/silicone emulsifying base.

WO03028690 describes solid water/oil emulsions as moisturising, anti-wrinkle cleansing compositions comprising BHT, tocopherol and retinol derivatives mixed powder comprising polymethylsilsesquioxane and coated silica, PMMA particles vinyl dimethicone as emulsifier but no polymethylmethacrylate encapsulation and hydrophobic polymers in the powder.

WO9922696 describes anhydrous makeup compositions for topical application to the skin. The composition comprise a silicone gel comprising an organopolysiloxane elastomer dispersed in dimethicone, a silicone-oil base, powders and antioxidants, tocopherol and/or retinol derivatives. This document does not disclose the specific anti-wrinkle powder with PMMA encapsulation and a hydrophobic polymer.

WO9716157 describes a lipstick composition comprising powders, pigments and liquid ingredients such as antioxidant BHA, anti-wrinkle retinyl palmitate, and emulsfier cyclomethicone, however, lacks the mixed powder.

However, these have disadvantages that retinol and its derivatives cannot be completely stable on account of their very high instability, and special apparatuses or closed containers is required upon the production.

Further, in such an emulsion-type cosmetics, since water is highly compatible with the powder containing a large amount of the liquid phase ingredient, the powder readily coagulates. As a result, there are a number of disadvantages that they are difficult to formulate into a powder and are very tacky upon use. Particularly, titer retention is reduced, and discoloration occurs and unpleasant odors are likely to develop, so that liquid phase-containing powder is not suitable for formulation as emulsion-type cosmetics.

### SUMMARY OF THE INVENTION

Therefore, the present invention has been made in view of the above-mentioned problems, and it is an object of the present invention to provide powder-type cosmetic compositions containing a large amount of liquid phase ingredient, which can impart anti-wrinkling effect to the skin, mask skin troubles, stabilize an anti-wrinkling ingredient and be easily penetrated into the skin.

### DETAILED DESCRIPTION OF THE PREFERRED EMBODIMENT

The present invention relates to liquid phase ingredient-containing powder-type cosmetic compositions which comprise: an anti-wrinkling ingredient-containing powder obtainable by dissolving an anti-wrinkling ingredient in a solvent, encapsulating the solution by a crosslinked polymethylmethacrylate (PMMA), and surface-treating the capsule using a hydrophobic polymer; a mixed powder including one or more powders selected from the group consisting of polymethylsilsesquioxane having an average particle size of 3.5 µm and a specific surface area of 110-150 m²/g, HDI/Trimehylol hexyllatactone and silica cross polymer having an average particle size of 3.7 µm and a specific surface area of 150-180 m²/g, laurylmethacrylate/glycoldimethacrylate cross polymer having an average particle size of 25 µm and a specific surface area of 100-300 m²/g, and an amorphous silica having an average particle size of 5.0 µm and an oil absorption of 0.3-4 ml/g; and a polyol/silicone emulsifying base for stabilizing a formulation and the active ingredient.

Specifically, the powder-type cosmetic compositions according to the present invention are produced by the steps of dissolving an anti-wrinkling ingredient in an oily solvent, encapsulating the solution with a crosslinked polymethylmethacrylate (PMMA), coating the capsule with a hydrophobic polymer, and finally applying the coated powder into a powder containing a large amount of liquid phase ingredient.

The present inventor has continuously and intensively studied the powder-type cosmetic compositions which can impart anti-wrinkling effect to the skin, mask skin troubles, stabilize anti-wrinkling ingredient and easily penetrate into the skin, and as a result, has developed a powder containing a large amount of liquid phase ingredient using an amorphous powder capable of effectively absorbing the liquid phase ingredient. Further, the present inventor produced an anti-wrinkling ingredient-containing powder by dissolving an anti-wrinkling ingredient in a solvent such as ester, encapsulating the solution with a crosslinked polymethylmethacrylate (PMMA) powder, and coating the capsule with a hydrophobic polymer to stabilize the powder. The anti-wrinkling ingredient-containing powder thus produced releases the anti-wrinkling ingredient slowly during the use of the cosmetic compositions containing such powder.

The cosmetic compositions of the present invention contain 1.0-10.0% by weight of the anti-wrinkling ingredient.

According to a preferred embodiment of the invention, there is provided a powder in which the anti-wrinkling ingredient is stabilized and its titer is well preserved.

First, an anti-wrinkling ingredient such as retinol, retinoic acid, retinyl aldehyde, retinyl acetate, retinyl propionate, retinyl palmitate, retinyl oleate, retinyl linoleate, etc., and at least one antioxidant selected from the group consisting of tocopherol, tocopheryl ester such as tocopheryl acetate and tocopheryl linoleate, unsaturated fatty acids and derivatives thereof, polyunsaturated fatty acids and derivatives thereof (vitamin F), BHT[2,6 bis(1,1-dimethylethyl)-4-methoxyphenol], BHA[butylated hydroxyanisole] and oxygenase together with a colorant in an amount of 1-30% by weight of the encapsulated powder to be produced are dissolved in an oily solvent. Subsequently, the solution is encapsulated with a crosslinked polymethylmethacrylate (PMMA) and then lyophilized to control the particle size of the capsule, depending on formulation properties.

Examples of the oily solvents which may be used in the invention include hexylaurate, squalane, trioctyldodecylcitrate, etc. And, all kinds of oily materials such as vegetable oils, animal oils, synthetic oils, mineral oils, etc., can be used, provided that their liquid states can be maintained at room temperature.

For producing the anti-wrinkling ingredient-containing capsule having excellent usability and stability in the final cosmetic compositions to be produced, the surface of the capsule is hydrophobically treated. When the hydrophobicity of the capsule is poor, the formulation into powder is difficult. When the hydrophobicity of the capsule is high, the usability of the final cosmetic compositions to be produced is very poor and the formulation is also very difficult to carry out. Therefore, the hydrophobic treatment of the capsule is a highly important process in the present invention.

Examples of the hydrophobizing compounds which are commonly used in the prior art include silicone-based compounds such as methicone, dimethicone, cyclomethicone, phenyltrimethicone, etc., fluorine compounds, amino acid salts, etc. When the capsule surface is treated using these compounds, its hydrophobicity is satisfactory but its stability and usability in the final cosmetic compositions to be produced are poor.

To overcome the above problems, a mixture of cyclomethicone, dimethicone, vinyl dimethicone cross polymer and cetyl dimethicone copolyol is used for the hydrophobic surface treatment of the capsule in the present invention.

When surface-treating the capsule, an optimal content of the hydrophobizing compounds is from 0.5 to 5% by weight, preferably from 0.5 to 2.0% by weight of the encapsulated powder. When the content is less than 0.5% by weight, the hydrophobicity is poor so that the anti-wrinkling ingredient is released into the aqueous solvent and also the usability in the final cosmetic compositions becomes poor. When the content is more than 2.0% by weight, the stability of the capsule is high but the affinity for water is little or none so that the formulation becomes difficult and the usability of the final cosmetic compositions is poor.

The relative weight ratio of the silicone-based compounds, i.e. cyclomethicone, dimethicone, vinyl dimethicone cross polymer and cetyl dimethicone copolyol is 20-40: 30-60: 3-10: 0.1-2.0, based on the total weight of the hydrophobizing compounds.

According to another preferred embodiment of the invention, there is provided a powder containing a large amount of liquid phase ingredient.

In order to effectively absorb water, a mixed powder including one or more powders selected from the group consisting of polymethylsilsesquioxane having an average particle size of 3.5 µm and a specific surface area of 110-150 m²/g, HDI/Trimethylol hexyllatactone and silica cross polymer having an average particle size of 3.7 µm and a specific surface area of 150-180 m²/g, laurylmethacrylate/glycoldimethacrylate having an average particle size of 25 µm and a specific surface area of 100-300 m²/g, and an amorphous silica having an average particle size of 5.0 µm is used.

The mixed powder can be used in an amount ranging from 5 to 15% by weight, based on the total weight of the final cosmetic compositions to be produced.

In order to impart other colors to the powder, iron oxides, titanium dioxide or pearl of an appropriate amount can be additionally blended.

According to a third preferred embodiment of the invention, there is provided a powder containing a large amount of liquid phase ingredient and the stabilized anti-wrinkling ingredient.

When the powder is mixed with the liquid phase ingredient, coagulation occurs and their compatibility decreases.

To solve these problems, polyol (aqueous ingredient)/silicone emulsifying base is used in the present invention. The emulsifying base enables the formulation to be easily made and stabilize the anti-wrinkling ingredient. Also, the emulsifying base makes the initial dispersion of the powder easy and enhances adhesiveness and durability of the aqueous ingredient when applied to the skin. Further, the emulsifying base prevents the powder from being dispersed by air currents.

Examples of the polyol which may be employed in the emulsifying base include glycerin, propylene glycol, mannan, soyful, etc., and examples of the silicone include cyclomethicone, dimethicone, vinyl dimethicone silicone powder, cetyl dimethicone copolyol, etc.

The polyol and silicone can be present in an amount of from 5 to 20% and from 2 to 10% by weight of the total weight of the emulsifying base, respectively.

Purified water can be added to the emulsifying base in an amount of from 70 to 93% by weight of the total weight of the emulsifying base. In the end, the liquid phase ingredient such as water, glycerin, etc., is present in an amount of from 50 to 80% by weight, based on the final powder-type cosmetic compositions.

The following examples are provided for purposes of illustration only and are not intended to limit the present invention.

### [Preparative Example]

First, a capsule was produced by dissolving anti-wrinkling ingredients, antioxidants and other effective ingredients of the following % by weight listed in Table 1 in an oily sovent, and then encapsulating the solution with a crosslinked polymethylmethacrylate (PMMA).

**[Table 1]**

| No | Ingredients | Sample 1 | Sample 2 |
|---|---|---|---|
| 1 | Crosslinked polymethylmethacrylate (PMMA) | 92.7 | 81.7 |
| 2 | Retinol | 1.0 | 8.0 |
| 3 | Retinyl palmitate | 1.0 | - |
| 4 | Hexylaurate | 5.0 | 10.0 |
| 5 | BHT | 0.2 | 0.2 |
| 6 | Methyl paraffin | 0.1 | 0.1 |

### (Procedure)

1) Ingredient 1 was charged into a high speed Henshel mixer, and mixed at 1000 rpm.
2) Ingredients 2 to 6 were dissolved using a high speed Ultra mixer at room temperature.
3) The mixture obtained in 1) above was slowly added while mixing the solution obtained in 2) above at 1500 rpm, and then lyophilized.

Next, the capsule obtained thus was hydrophobically surface-treated with the ingredients of the following % by weight listed in Table 2.

**[Table 2]**

| No. | Ingredients | Sample 3 | Sample 4 |
|---|---|---|---|
| 1 | Sample 1 | 98 | - |
| 2 | Sample 2 | - | 97.5 |
| 3 | Cyclomethicone | 0.50 | 0.40 |
| 4 | Dimethicone | 1.20 | 1.33 |
| 5 | Vinyl dimethicone cross polymer | 0.20 | 0.20 |
| 6 | Cetyl dimethicone copolyol | 0.10 | 0.07 |

### (Procedure)

1) Ingredients 3-6 were mixed using a high speed Ultra mixer at 3500 rpm for 20 minutes.
2) Ingredients 1 and 2 were charged into a high speed Henshel mixer and mixed at 1000 rpm. Ingredient 1 was sprayed thereon and then lyophilized.

A polyol/silicone emulsifying base was produced in accordance with the ingredients of the following % by weight listed in Table 3.

**[Table 3]**

| No. | Ingredients | Sample 5 | Sample 6 |
|---|---|---|---|
| 1 | Cetyl dimethicone copolyol | 0.4 | 0.5 |
| 2 | Dimethicone (6 cs) | 10.0 | 5.0 |
| 3 | Dimethicone (8 cs) | - | 5.0 |
| 4 | Cyclomethicone | 3.0 | 5.0 |
| 5 | Lauryl dimethicone copolyol | 0.3 | 0.5 |
| 6 | Purified water | To 100 | To 100 |
| 7 | Glycerin | 10.0 | 5.0 |
| 8 | 1,3-butylene glycol | 5.0 | 10.0 |
| 9 | Konjak | 0.5 | 0.5 |
| 10 | D,L-panthenol | 0.1 | 0.1 |
| 11 | Preservative | q.s | q.s |
| 12 | Flavoring agent | q.s | q.s |

### (Procedure)

Ingredients 1-5 were heated to 70-75°C to form an oily phase. Ingredients 6-11 were heated to 70-75°C and ingredient 11 was added thereto to form an aqueous phase. The aqueous phase was slowly added to the oily phase, and emulsified using Homo Rpm 3000 for 10 minutes. Ingredient 12 was added to the emulsion, emulsified for 1 minute and then cooled to 30°C.

Finally, the powder-type cosmetic compositions according to the present invention were prepared with ingredients of the following % by weight listed in Table 4, including the anti-wrinkling ingredients-containing powder and the emulsifying base produced above.

**[Table 4]**

| No. | Ingredients | Exam. 1 | Exam. 2 | Exam. 3 | Comp. Exam 1 | Comp. Exam 2 |
|---|---|---|---|---|---|---|
| 1 | Sample 1 | 3.0 | - | - | - | - |
| 2 | Sample 3 | - | 3.0 | - | - | - |
| 3 | polymethylsilsesquioxane | 5.0 | 3.0 | 5.0 | 5.0 | - |
| 4 | Licorice extract (Squalane 0.10%) | - | - | - | - | 0.5 |
| 5 | HDI/Trimethylol hexyllatactone/silica cross polymer (and) Silica | 5.0 | 12.0 | 5.0 | 5.0 | - |
| 6 | Silica (hydrophobic) | - | - | - | - | 10.0 |
| 7 | PMMA (retinyl palmitate) | - | - | - | 3.0 | 3.0 |
| 8 | Purified water | - | - | - | To 100 | To 100 |
| 9 | Condensed glycerin | - | - | - | 8.3 | - |
| 10 | 1,3-butylen glycol | - | - | - | 4.15 | - |
| 11 | Preservative | - | - | - | q.s | q.s |
| 12 | Sample 5 | To 100 | - | To 100 | - | - |
| 13 | Sample 6 | - | To 100 | - | - | - |
| 14 | Sample 3 | - | - | 3.0 | - | - |
| 15 | Retinal (O/W emulsion 0.1%) | - | - | - | - | 0.5 |
| 16 | Polyphenol | 0.2 | 0.2 | 0.2 | 0.2 | 0.2 |

### (Procedure)

1) Ingredients 1-6 were charged into a Henshel mixer, and mixed.
2) Ingredients 8-16 were homogeneously mixed using AGI mixer, and sprayed onto the mixture obtained in 1) above to obtain a formulation.

### [Experimental Example 1]

The physical state of the formulations produced in the above Examples and Comparative Examples was evaluated by a sensory test. The stability of the compositions was evaluated in terms of discoloration and malodor and titer retention. The physical state of the powder was evaluated by a sensory test, and the results are presented in the following Table 5. The discoloration/malodor tests were performed after storing the compositions at 45°C for 4 weeks, and titer retention test was performed by measuring the absorbance using an HPLC (325 nm) immediately after their productions and after storing the compositions at 45°C for 1 month, respectively. The results are listed in the following Table 6, respectively.

**[Table 5]**

| Physical state of powders | | | | | |
|---|---|---|---|---|---|
| | Exam. 1 | Exam. 2 | Exam. 3 | Comp. Exam. 1 | Comp. Exam. 2 |
| 0 day | a | a | a | c | c |
| 1 month (45°C) | b | a | a | d | d |

| | | | | | |
|---|---|---|---|---|---|
| a: ≥ 95% excellent, b: ≥ 80% good, c: ≥ 70% average, d: < 50% poor | | | | | |

**[Table 6]**

| | Exam. 1 | | Exam. 2 | | Exam. 3 | | Comp. Exam. 1 | | Comp. Exam. 2 | |
|---|---|---|---|---|---|---|---|---|---|---|
| Discoloration/malodor (45°C,4weeks) | b | | a | | a | | d | | d | |
| Titer retention (%) | 0 day | 1 mon | 0 day | 1 mon | 0 day | 1 mon | 0 day | 1 mon | 0 day | 1 mon |
| | 98.4 | 97.5 | 99.2 | 96.6 | 99.5 | 96.3 | 98.4 | 36.5 | 98.6 | 50.6 |

| | | | | | | | | | | |
|---|---|---|---|---|---|---|---|---|---|---|
| a: ≥ 95% excellent, b: ≥ 80% good, c: ≥ 70% average, d: < 50% poor. | | | | | | | | | | |

It has been proven from the Tables 5 and 6 that the compositions according to the present invention can be more feasibly formulated into powder and stored longer than those of the Comparative Examples. It is assumed that the coagulation of powder is inhibited and stability at high temperatures is excellent by treating the surface of the powder with silicone mixture and using polyol/silicone emulsifying base.

Also, the compositions of the Examples were superior to those of the Comparative Examples in terms of titer retention after storing the compositions at 45°C for 1 month. This result indicates that the emulsified films of the powder are more stable, due to treating the surface of the powder with silicone mixture and using polyol/silicone emulsifying base.

### [Experimental Example 2]

The powder-type cosmetic compositions according to the present invention were evaluated by measuring skin irritation and liquefied states upon their use.

The measurement of skin irritations was carried out in a sensory test by adhering patches to 20 healthy women aged 25-35 and then removing the patches after 24 hours. When no irritation to the skin was observed, the composition was scored as "0". Severe irritation was scored as "5", and the scores are expressed as averages. The results are listed in the following Table 7. Also, after the powder-type cosmetic compositions of the Examples and Comparative Examples were applied to the test subjects once a day for 1 month, the sensory tests for usability and liquefied state during the use of the compositions were carried out. The results are listed in the following Table 8.

**[Table 7]**

| | Exam. 1 | Exam. 2 | Exam. 3 | Comp. Exam. 1 | Comp. Exam. 2 |
|---|---|---|---|---|---|
| Skin irritation | 0.7 | 1.0 | 0.9 | 3.24 | 2.35 |

**[Table 8]**

| | Exam. 1 | Exam. 2 | Exam. 3 | Comp. Exam. 1 | Comp. Exam. 2 |
|---|---|---|---|---|---|
| Usability | b | a | a | d | e |
| Liquefied state | b | a | a | c | c |

| | | | | | |
|---|---|---|---|---|---|
| a: excellent, b: 80% good, c: 70% average, d: below average, e: poor | | | | | |

As can be seen from the Table 7, the compositions of the Examples exhibited superior stability to the Comparative Examples. From this observation, it is assumed that the anti-wrinkling ingredient, e.g., letinol, encapsulated in the capsule is slowly released to minimize skin irritation.

As can be seen from the Table 8, the compositions of Examples exhibited more excellent usability and liquefied state upon use than those of Comparative Examples. This result shows that since powder-type cosmetic compositions of the present invention contain the liquid phase ingredient and polyol/silicone emulsifying base, they have a pleasant feel upon use in spite of containing powder.

### [Experimental Example 3]

In order to evaluate the anti-wrinkling effect of the powder-type cosmetic compositions according to the present invention, the change in skin elasticity upon using the cosmetic compositions of Examples and Comparative Examples produced in accordance with the above Table 4 was tested.

Two compositions selected from the cosmetic compositions of the Examples and Comparative Examples were respectively applied on each of both sides of the faces of 20 women aged 25-35 as test subjects twice a day for 2 months, under indoor and outdoor environmental conditions of a temperature of 24-26°C and at a relative humidity of 38-40%. After 2 months, the change in skin elasticity was evaluated by means of Cutometer SEM 474. When no change in skin elasticity was observed, the composition was scored as "0". A significant increase in skin elasticity was scored as "5", and the scores are expressed as averages. The results are listed in the following Table 9.

**[Table 9]**

| | Exam. 1 | Exam. 2 | Exam. 3 | Comp. Exam. 1 | Comp. Exam. 2 |
|---|---|---|---|---|---|
| Skin elasticity | 4.7 | 4.4 | 4.6 | 1.25 | 3.35 |

As can be seen from the Table 9, the compositions of the Examples exhibited superior anti-wrinkling effect to the Comparative Examples. This result shows that the encapsulated anti-wrinkling ingredient effectively penetrates into the skin.

As described above, the powder-type cosmetic compositions according to the present invention can impart anti-wrinkling effect to the skin, mask skin troubles, stabilize anti-wrinkling ingredients and easily penetrate into the skin. At this time, the anti-wrinkling ingredient encapsulated in the capsule is slowly released to minimize skin irritation.

Further, the compositions according to the present invention can maintain their titer for a long time and prevent the powder from being dispersed by air currents.

## Claims

1. A powder-type cosmetic composition comprising:
an anti-wrinkling ingredient-containing powder obtainable by dissolving an anti-wrinkling ingredient and an antioxidant in a solvent, encapsulating the solution by a crosslinked polymethylmethacrylate (PMMA), and surface-treating the capsule with a hydrophobic polymer;
a mixed powder including one or more powders selected from the group consisting of polymethylsilsesquioxane having an average particle size of 3.5 µm and a specific surface area of 110-150 m²/g, HDI/Trimehylol hexyllatactone and silica cross polymer having an average particle size of 3.7 µm and a specific surface area of 150-180 m²/g, laurylmethacrylate/glycoldimethacrylate cross polymer having an average particle size of 25 µm and a specific surface area of 100-300 m²/g, and an amorphous silica having an average particle size of 5.0 µm and an oil absorption of 0.3-4 ml/g; and
a polyol/silicone emulsifying base.

2. The cosmetic composition as set forth in claim 1, wherein the anti-wrinkling ingredient is at least one selected from the group consisting of Retinol (vitamin A alcohol) and its derivatives include retinal, retinoic acid, retinyl aldehyde, retinyl acetate, retinyl propionate, retinyl palmitate, retinyl oleate and retinyl esters.

3. The cosmetic composition as set forth in claim 1 or 2, wherein the anti-wrinkling ingredient is present in an amount 1.0-10.0% by weight of the final cosmetic composition.

4. The cosmetic composition as set forth in claim 1, wherein the solvent is an oily solvent selected from hexylaurate, squalane, trioctyldodecylcitrate, vegetable oils, animal oils, synthetic oils and mineral oils.

5. The cosmetic composition as set forth in claim 1, wherein the antioxidant is selected from the group consisting of tocopherol, tocopheryl acetate, tocopheryl linoleate, unsaturated fatty acids and derivatives thereof, polyunsaturated fatty acids and derivatives thereof, BHT[2,6 bis(1,1-dimethylethyl)-4-methoxyphenol], BHA[butylated hydroxyanisole] and oxygenase, and is present in an amount ranging from 0.05 to 2.0% by weight of total weight of the final cosmetic composition.

6. The cosmetic composition as set forth in claim 1, wherein the hydrophobic polymer is a mixture of at least one polymer selected from cyclomethicone, dimethicone, vinyl dimethicone cross polymer and cetyl dimethicone copolyol.

7. The cosmetic composition as set forth in claim 1 or 6, wherein the hydrophobic polymer is present in an amount of from 0.5 to 5% by weight of the encapsulated powder.

8. The cosmetic composition as set forth in claim 1 or 6, wherein the relative weight ratio of cyclomethicone, dimethicone, vinyl dimethicone cross polymer and cetyl dimethicone copolyol is 20-40: 30-60: 3-10: 0.1-2.0, based on the total weight thereof.

9. The cosmetic composition as set forth in claim 1, wherein the mixed powder is present in an amount ranging from 5 to 15% by weight of the total weight of the final cosmetic composition.

10. The cosmetic composition as set forth in claim 1, wherein the polyol of the polyol/silicone emulsifying base is selected from glycerin propylene glycol, mannan and soyful, and the silicone of the polyol/silicone emulsifying base is selected from cyclomethicone, dimethicone, vinyl dimethicone silicone powder and cetyl dimethicone copolyol.

11. The cosmetic composition as set forth in claim 1 or 10, wherein the polyol and silicone of the polyol/silicone emulsifying base is present in an amount of from 5 to 20% and from 2 to 10% by weight of the total weight of the emulsifying base, respectively, and purified water is added to the emulsifying base in an amount of from 70 to 93% by weight of the total weight of the emulsifying base.

12. The cosmetic composition as set forth in claim 1, wherein liquid phase ingredient is present in an amount of from 50 to 80% by weight, based on the final cosmetic composition.

## Patentansprüche

1. Kosmetische Zusammensetzung vom Pulvertyp, umfassend:
ein einen Antifaltenwirkstoff enthaltendes Pulver, das erhalten werden kann, indem ein Antifaltenwirkstoff und ein Antioxidans in einem Lösungsmittel gelöst werden, die Lösung durch ein vernetztes Polymethylmethacrylat (PMMA) eingekapselt wird und die Kapsel mit einem hydrophoben Polymer oberflächenbehandelt wird;
ein gemischtes Pulver, das ein oder mehrere Pulver enthält, ausgewählt aus der Gruppe bestehend aus Polymethylsilylsesquioxan mit einer durchschnittlichen Teilchengröße von 3,5 µm und einer spezifischen Oberfläche von 110 - 150 m²/g, einem Kreuzpolymer von HDI/Trimethylolhexyllacton und Kieselsäure mit einer durchschnittlichen Teilchengröße von 3,7 µm und einer spezifischen Oberfläche von 150 - 180 m²/g, einem Laurylmethacrylat/Glykoldimethacrylat-Kreuzpolymer mit einer durchschnittlichen Teilchengröße von 25 µm und einer spezifischen Oberfläche von 100 - 300 m²/g und einer amorphen Kieselsäure mit einer durchschnittlichen Teilchengröße von 5,0 µm und einer Ölabsorption von 0,3 - 4 ml/g; und
eine Polyol/Silikon-Emulsionsbasis.

2. Kosmetische Zusammensetzung nach Anspruch 1, wobei der Antifaltenwirkstoff mindestens einer der aus der Gruppe bestehend aus Retinol (Vitamin-A-Alkohol) und dessen Derivaten, einschließlich Retinal, Retinsäure, Retinylaldehyd, Retinylacetat, Retinylpropionat, Retinylpalmitat, Retinyloleat und Retinylestern, ausgewählten ist.

3. Kosmetische Zusammensetzung nach Anspruch 1 oder 2, wobei der Antifaltenwirkstoff in einer Menge von 1,0 - 10,0 Gew.-% der fertigen kosmetischen Zusammensetzung vorliegt.

4. Kosmetische Zusammensetzung nach Anspruch 1, wobei das Lösungsmittel ein öliges Lösungsmittel ist, das aus Hexyllaurat, Squalan, Trioctyldodecylcitrat, pflanzlichen Ölen, tierischen Ölen, synthetischen Ölen und Mineralölen ausgewählt ist.

5. Kosmetische Zusammensetzung nach Anspruch 1, wobei das Antioxidans aus der Gruppe bestehend aus Tocopherol, Tocopherolacetat, Tocopherollinoleat, ungesättigten Fettsäuren und Derivaten davon, mehrfach ungesättigten Fettsäuren und Derivaten davon, BHT [2,6-Bis-(1,1-dimethylethyl)-4-methoxyphenol], BHA [Butylhydroxyanisol] und Oxygenase ausgewählt ist und in einer Menge vorliegt, die von 0,05 bis 2,0 Gew.-% des Gesamtgewichts der fertigen kosmetischen Zusammensetzung reicht.

6. Kosmetische Zusammensetzung nach Anspruch 1, wobei das hydrophobe Polymer ein Gemisch von mindestens einem Polymer ist, ausgewählt aus Cyclomethicon, Dimethicon, Vinyldimethicon-Kreuzpolymer und Cetyldimethicon-Copolyol.

7. Kosmetische Zusammensetzung nach Anspruch 1 oder 6, wobei das hydrophobe Polymer in einer Menge von 0,5 bis 5 Gew.-% des eingekapselten Pulvers vorliegt.

8. Kosmetische Zusammensetzung nach Anspruch 1 oder 6, wobei das relative Gewichtsverhältnis von Cyclomethicon, Dimethicon, Vinyldimethicon-Kreuzpolymer und Cetyldimethicon-Copolyol 20-40:30-60:3-10:0,1-2,0, bezogen auf das Gesamtgewicht dieser, beträgt.

9. Kosmetische Zusammensetzung nach Anspruch 1, wobei das gemischte Pulver in einer Menge vorliegt, die von 5 bis 15 Gew.-% des Gesamtgewichts der fertigen kosmetischen Zusammensetzung reicht.

10. Kosmetische Zusammensetzung nach Anspruch 1, wobei das Polyol der Polyol/Silikon-Emulsionsbasis aus Glycerinpropylenglykol, Mannan und Soyful ausgewählt ist und das Silikon der Polyol/Silikon-Emulsionsbasis aus Cyclomethicon, Dimethicon, Vinyldimethicon-Silikonpulver und Cetyldimethicon-Copolyol ausgewählt ist.

11. Kosmetische Zusammensetzung nach Anspruch 1 oder 10, wobei das Polyol und das Silikon der Polyol/Silikon-Emulsionsbasis in einer Menge von 5 bis 20 Gew.-% bzw. von 2 bis 10 Gew.-% des Gesamtgewichts der Emulsionsbasis vorliegen und destilliertes Wasser der Emulsionsbasis in einer Menge von 70 bis 93 Gew.-% des Gesamtgewichts der Emulsionsbasis zugeben wird.

12. Kosmetische Zusammensetzung nach Anspruch 1, wobei der Flüssigphasenbestandteil in einer Menge von 50 bis 80 Gew.-%, bezogen auf die fertige kosmetische Zusammensetzung, vorliegt.

## Revendications

1. Composition cosmétique de type poudre, comprenant :
une poudre contenant un ingrédient antirides que l'on peut obtenir en dissolvant un ingrédient antirides et un antioxydant dans un solvant, en encapsulant la solution par un poly(méthacrylate de méthyle) (PMMA) réticulé, et en effectuant un traitement de surface de la capsule avec un polymère hydrophobe ;
une poudre mixte comprenant une ou plusieurs poudres choisies parmi le groupe constitué par le poly(méthyl silsesquioxane) ayant une granulométrie moyenne de 3,5 µm et une surface spécifique de 110 à 150 m²/g, un polymère réticulé de HDI/Triméthylol hexyllactone et de silice ayant une granulométrie moyenne de 3,7 µm et une surface spécifique de 150 à 180 m²/g, un polymère réticulé de méthacrylate de lauryle/diméthacrylate de glycol ayant une granulométrie moyenne de 25 µm et une surface spécifique de 100 à 300 m²/g, et une silice amorphe ayant une granulométrie moyenne de 5,0 µm et une absorption d'huile de 0,3 à 4 mL/g ; et
une base émulsifiante de polyol/silicone.

2. Composition cosmétique selon la revendication 1, dans laquelle l'ingrédient antirides est au moins un élément choisi dans le groupe constitué par le rétinol (alcool de vitamine A) et ses dérivés incluant le rétinal, l'acide rétinoïque, le rétinyl aldéhyde, l'acétate de rétinyle, le propionate de rétinyle, le palmitate de rétinyle, l'oléate de rétinyle et les esters de rétinyle.

3. Composition cosmétique selon la revendication 1 ou 2, dans laquelle l'ingrédient antirides est présent dans une quantité allant de 1,0 à 10,0 % en poids de la composition cosmétique finale.

4. Composition cosmétique selon la revendication 1, dans laquelle le solvant est un solvant huileux choisi parmi le laurate d'hexyle, le squalane, le citrate de trioctyldodécyle, les huiles végétales, les huiles animales, les huiles synthétiques et les huiles minérales.

5. Composition cosmétique selon la revendication 1, dans laquelle l'antioxydant est choisi parmi le groupe constitué par le tocophérol, l'acétate de tocophéryle, le linoléate de tocophéryle, les acides gras insaturés et leurs dérivés, les acides gras polyinsaturés et leurs dérivés, le BHT [2,6 bis(1,1-diméthyléthyl)-4-méthoxyphénol], le BHA [hydroxyanisole butylé] et l'oxygénase, et est présent dans une quantité allant de 0,05 à 2,0 % en poids du poids total de la composition cosmétique finale.

6. Composition cosmétique selon la revendication 1, dans laquelle le polymère hydrophobe est un mélange d'au moins un polymère choisi parmi la cyclométhicone, la diméthicone, un polymère réticulé de vinyl diméthicone et le cétyl diméthicone copolyol.

7. Composition cosmétique selon la revendication 1 ou 6, dans laquelle le polymère hydrophobe est présent dans une quantité allant de 0,5 à 5 % en poids de la poudre encapsulée.

8. Composition cosmétique selon la revendication 1 ou 6, dans laquelle le rapport en poids relatif de la cyclométhicone, la diméthicone, le polymère réticulé de vinyl diméthicone et le cétyl diméthicone copolyol est de 20 à 40 :30 à 60 :3 à 10 :0,1 à 2,0 par rapport à leur poids total.

9. Composition cosmétique selon la revendication 1, dans laquelle la poudre mixte est présente dans une quantité allant de 5 à 15 % en poids du poids total de la composition cosmétique finale.

10. Composition cosmétique selon la revendication 1, dans laquelle le polyol de la base émulsifiante de polyol/silicone est choisi parmi le propylène glycol de glycérine, la mannane et le « soyful », et le silicone de la base émulsifiante de polyol/silicone est choisi parmi la cyclométhicone, la diméthicone, la poudre de vinyl diméthicone silicone et le cétyl diméthicone copolyol.

11. Composition cosmétique selon la revendication 1 ou 10, dans laquelle le polyol et le silicone de la base émulsifiante de polyol/silicone sont présents dans des quantités de 5 à 20 % et de 2 à 10 % en poids du poids total de la base émulsifiante, respectivement, et de l'eau purifiée est ajoutée à la base émulsifiante dans une quantité allant de 70 à 93 % en poids du poids total de la base émulsifiante.

12. Composition cosmétique selon la revendication 1, dans laquelle l'ingrédient en phase liquide est présent dans une quantité de 50 à 80 % en poids, par rapport à la composition cosmétique finale.
